(19) Europäisches Patentamt
European Patent Office
Office européen des brevets

(11) Numéro de publication: **0 342 068 A1**

(12) # DEMANDE DE BREVET EUROPEEN

(21) Numéro de dépôt: **89400949.7**

(22) Date de dépôt: **06.04.89**

(51) Int. Cl.[4]: **G 01 N 33/543**
G 01 N 33/545

(30) Priorité: **08.04.88 FR 8804685**

(43) Date de publication de la demande:
**15.11.89 Bulletin 89/46**

(84) Etats contractants désignés:
**AT BE CH DE ES FR GB GR IT LI LU NL SE**

(71) Demandeur: **SOCIETE D'APPLICATIONS PHARMACEUTIQUES ET BIOLOGIQUES HOECHST-BEHRING**
**260 avenue Napoléon Bonaparte**
**F-92500 Rueil Malmaison (FR)**

(72) Inventeur: **Deschamps, Françoise**
**20 Rue de la Madeleine**
**F-61300 L'Aigle (FR)**

**Tobo, Jean**
**Le Plessis**
**F-61270 Aube (FR)**

**Volle, Pierre Jean**
**141 Boulevard Péreire**
**F-75017 Paris (FR)**

(74) Mandataire: **Orès, Bernard et al**
**Cabinet ORES 6, Avenue de Messine**
**F-75008 Paris (FR)**

(54) **Support solide pour diagnostic immunologique, son procédé de traitement et application au diagnostic immunologique.**

(57) Support solide pour diagnostic immunologique, son procédé de traitement et son application au diagnostic immunologique.

Le support solide à usage biologique présente une polarité de surface modifiée par la fixation sur ladite surface d'un composé chimique entraînant la formation de radicaux libres et/ou de chaînes carbonées aliphatiques dérivées de structures cycliques métastables, ledit composé chimique étant de formule I,

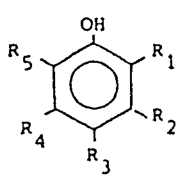

dans laquelle :
$R_1$ peut être un atome d'hydrogène, un groupe hydroxyle, un groupe $NO_2$, un halogène, un groupe alkyl inférieur comprenant 1 à 4 atomes de carbone, une amine ;
$R_2$ peut être un atome d'hydrogène, un groupe hydroxyle, un groupe $NO_2$, un halogène, un groupe alkyl inférieur comprenant 1 à 4 atomes de carbone, une amine ;
$R_3$ peut être un atome d'hydrogène, un groupe hydroxyle, un groupe $NO_2$, un halogène, un groupe alkyl inférieur comprenant 1 à 4 atomes de carbone, une amine, un groupe NO ;
$R_4$ peut être un atome d'hydrogène, un groupe $NO_2$, un halogène, un groupe hydroxyle ;
$R_5$ peut être un atome d'hydrogène, un groupe $NO_2$, un halogène, un groupe alkyl inférieur comprenant 1 à 4 atomes de carbone, un groupe hydroxyle.

EP 0 342 068 A1

**Description**

## SUPPORT SOLIDE POUR DIAGNOSTIC IMMUNOLOGIQUE, SON PROCEDE DE TRAITEMENT ET APPLICATION AU DIAGNOSTIC IMMUNOLOGIQUE

La présente invention est relative à un support solide pour diagnostic immunologique, à son procédé de traitement et à l'application au diagnostic immunologique.

Conformément à l'Art antérieur, des anticorps ou des antigènes peuvent être fixés sur un support solide, notamment en polystyrène, pour la mise en évidence d'antigènes, d'anticorps ou de micro-organismes, éventuellement présents dans un milieu liquide, en réalisant la liaison de l'antigène, de l'anticorps ou du micro-organisme à détecter avec l'anticorps ou l'antigène fixé sur le support solide ; la lecture de la réaction se fait généralement par comptage d'une radioactivité, mesure d'une activité enzymatique ou mesure physique.

Toutefois, les supports proposés dans l'Art antérieur présentent un certain nombre d'inconvénients et notamment ils fournissent une grande dispersion des résultats lors de la révélation de la réaction, ce qui ne permet pas une bonne reproductibilité, ni une bonne répétabilité des tests diagnostics.

Afin d'améliorer la fixation de l'antigène ou de l'anticorps sur lesdits supports solides, on a proposé un traitement par irradiation du support, de manière à obtenir de meilleurs résultats d'enduction et notamment d'adsorption de l'anticorps ou de l'antigène à la surface du support solide. En particulier, la Demande de Brevet européen n° 61 167 au nom de BEHRINGWERKE, décrit un procédé de traitement de support par irradiation au moyen de rayons gamma ; ce procédé améliore l'adsorption d'un ligand ou anti-ligand, à sa surface, dans le cadre d'une réaction immunologique.

Néanmoins, les techniques industrielles d'irradiation ne permettent pas d'effectuer un traitement homogène du support solide à des coûts peu onéreux.

La présente invention s'est, en conséquence, donné pour but de pourvoir à un nouveau support solide, à usage de diagnostic immunologique, qui répond mieux aux nécessités de la pratique que les moyens utilisés jusqu'à présent, notamment en ce qui concerne le coût, la reproductibilité et la répétabilité des tests effectués en série.

C'est également un but de l'invention de fournir un procédé de traitement desdits supports solides.

C'est, en outre, un but de l'invention d'appliquer lesdits supports traités à des tests diagnostics immunologiques.

La présente invention a pour objet un support solide à usage biologique, caractérisé en ce que ledit support solide présente une polarité de surface modifiée par la fixation sur ladite surface d'un composé chimique entraînant la formation de radicaux libres et/ou de chaînes carbonées aliphatiques dérivées de structures cycliques métastables, ledit composé chimique étant de formule I,

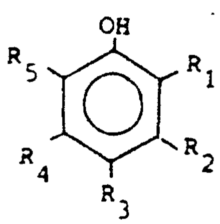

dans laquelle :

$R_1$ peut être un atome d'hydrogène, un groupe hydroxyle, un groupe $NO_2$ un halogène, un groupe alkyl inférieur comprenant 1 à 4 atomes de carbone, une amine ;

$R_2$ peut être un atome d'hydrogène, un groupe hydroxyle, un groupe $NO_2$, un halogène, un groupe alkyl inférieur comprenant 1 à 4 atomes de carbone, une amine ;

$R_3$ peut être un atome d'hydrogène, un groupe hydroxyle, un groupe $NO_2$, un halogène, un groupe alkyl inférieur comprenant 1 à 4 atomes de carbone, une amine, un groupe NO ;

$R_4$ peut être un atome d'hydrogène, un groupe $NO_2$, un halogène, un groupe hydroxyle ;

$R_5$ peut être un atome d'hydrogène, un groupe $NO_2$, un halogène, un groupe alkyl inférieur comprenant 1 à 4 atomes de carbone, un groupe hydroxyle.

Les composés chimiques conformes à la présente invention, peuvent notamment être le phénol, le 2,4-dinitrophénol, le 2,5-dinitrophénol, le 2,6-dinitrophénol, le 2,4,6-trinitrophénol, l'hydroquinone, le pyrocatechol, le pyrogallol, le resorcinol, le 2-bromophénol, le 3-bromophénol, le 4-bromophénol, le 2,4-dinitrorésorcinol, le dinitrocrésol, le 2-amino 4,6 dichlorophénol, l'iodophénol, le 2-aminophénol, le 3-aminophénol, le 4-aminophénol, le 1,2,4-benzenetriol, le chlorophénol, le méthylphénol, le 2-nitrophénol, le 3-nitrophénol, le 4-nitrophénol, le 4-nitrosophénol, le pentachlorophénol, le 2-amino 4,6-dinitrophénol, le 3,4,5,6-tétrabomo-o-crésol, le 2,4,6-tribromo-3-méthylphénol, le 2,4,6-tribromophénol, le 2,4,6-tri-chloro-3-méthylphénol, le 2,3,6-trichloro-4-méthylphénol, le 2,3,4-trichloro-6-méthylphénol, le 3,4,6-trichloro-2-nitrophénol, le 2,4,5-trichlorophénol, le 2,4,6-trichlorophénol.

Conformément à l'invention, lorsque le support solide est en plastique, il est choisi dans le groupe qui

comprend les polystyrènes, les copolymères styréniques, le chlorure de polyvinyle, le polyméthacrylate de méthyle.

Selon un mode de réalisation de la présente invention, lorsque le composé chimique est du 2,4,5-trichlorophénol ou du 2,4,6-trichlorophénol, le support est avantageusement un copolymère styrénique.

Selon un autre mode de réalisation de la présente invention, lorsque le composé chimique est du 2-bromophénol, du 3-bromophénol ou du 4-bromophénol, le support est avantageusement choisi dans le groupe qui comprend les copolymères styréniques et le chlorure de polyvinyle.

Selon encore un autre mode de réalisation de la présente invention, lorsque le composé chimique est du iodophénol, le support chimique est avantageusement choisi dans le groupe qui comprend les copolymères styréniques, le chlorure de polyvinyle et le polyméthacrylate de méthyle.

L'invention trouve application avantageuse pour tout support solide choisi dans le groupe qui comprend les plaques à cuves multiples isolées et isolables les unes des autres, les tubes, les tubes à essai, les tiges et les billes.

La présente invention a également pour objet un procédé de traitement d'un support solide à usage biologique, en particulier dans le cadre de dosages immunologiques, caractérisé en ce que ledit support est mis en contact avec un composé chimique de formule I :

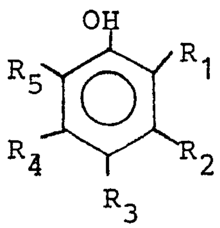

dans laquelle :

$R_1$ peut être un atome d'hydrogène, un groupe hydroxyle, un groupe $NO_2$, un halogène, un groupe alkyle inférieur comprenant 1 à 4 atomes de carbone, une amine ;

$R_2$ peut être un atome d'hydrogène, un groupe hydroxyle, un groupe $NO_2$, un halogène, un groupe alkyle inférieur comprenant 1 à 4 atomes de carbone, une amine ;

$R_3$ peut être un atome d'hydrogène, un groupe hydroxyle, un groupe $NO_2$, un halogène, un groupe alkyle inférieur comprenant 1 à 4 atomes de carbone, une amine, un groupe NO ;

$R_4$ peut être un atome d'hydrogène, un groupe $NO_2$, un halogène, un groupe hydroxyle ;

$R_5$ peut être un atome d'hydrogène, un groupe $NO_2$, un halogène, un groupe alkyle inférieur comprenant 1 à 4 atomes de carbone, un groupe hydroxyle.

Selon un mode de mise en oeuvre avantageux de l'invention, le support solide est mis en contact avec ledit composé chimique pendant un temps approprié et à une température appropriée, puis lavé et séché.

Selon une disposition préférée de ce mode de mise en oeuvre, le temps de contact est de 2 à 10 minutes.

Selon une autre disposition préférée de ce mode de mise en oeuvre, la température de traitement est comprise entre la température ambiante et 85°C.

Selon un autre mode de mise en oeuvre du procédé conforme à la présente invention, le support solide est soumis à la fois à un traitement par ledit composé chimique et à un traitement d'irradiation, notamment par les rayons gamma.

Selon une disposition avantageuse de ce mode de mise en oeuvre, le traitement d'irradiation est réalisé préalablement à la mise en contact avec le composé chimique.

Selon une autre disposition avantageuse de ce mode de mise en oeuvre, le traitement d'irradiation est réalisé postérieurement à la mise en contact avec le composé chimique.

Un tel traitement du support solide a l'avantage, lors de l'application desdits supports au dosage immunologique, de permettre l'enduction du support par un antigène ou un anticorps à fixer, de manière reproductible et contrôlée, de sorte que lesdits supports trouvent une application avantageuse dans les procédés de détection d'un anticorps, d'un antigène ou d'un micro-organisme éventuellement présents dans un milieu liquide, suivant lesquels procédés, un anticorps ou un antigène fixé sur un support solide traité conformément au procédé de l'invention, est incubé avec ledit milieu liquide, la révélation de la réaction antigène-anticorps étant réalisée, de manière connue par comptage d'une radio-activité, mesure d'une activité enzymatique ou mesure physique.

L'invention sera mieux comprise à l'aide du complément de description qui va suivre, qui se réfère à des exemples du procédé conforme à l'invention ainsi qu'à des études comparatives avec les supports du commerce.

Il doit être bien entendu, toutefois, que ces exemples sont donnés uniquement à titre d'illustration de l'objet de l'invention, dont ils ne constituent en aucune manière une limitation.

La mise en contact du composé chimique et du support peut avoir lieu sur les surfaces préférentielles de celui-ci, soit par remplissage, soit par immersion du support.

La mise en contact est de préférence de deux à dix minutes et à une température comprise entre la température ambiante et 85°C.

Le support solide est ensuite lavé, afin d'éliminer un possible dépôt puis séché soit à température ambiante, soit à l'étuve.

**Exemple 1 : Support solide en polystyrène traité au 2,4 dinitrophénol.**

Le polystyrène utilisé est notamment du polystyrène cristal pour applications médicales et biochimiques.

La concentration du 2,4 dinitrophénol est de 3 g/l dans l'eau ;
le temps de contact entre le support solide et le 2,4 dinitrophénol est de 5 minutes à 80°C ;
on met en contact le support solide en polystyrène avec le 2,4 dinitrophénol en immergeant ledit support dans un bain de 2,4 dinitrophénol ;
le support solide est ensuite lavé trois fois de suite à l'eau et séché à température ambiante.

**Exemple 2 : Support solide en polystyrène traité au 2,4,6 trinitrophénol.**

Le support est identique à celui de l'Exemple 1.

La concentration du 2,4,6 trinitrophénol est de 12 g/l dans l'eau ;
le temps de contact est de 15 minutes à température ambiant ;
le support solide est ensuite lavé à l'eau et séché à température ambiante.

**Exemple 3 : Comparaison entre un support solide conforme à l'invention, enduit avec des anticorps antiapolipoprotéines B et d'un support du commerce, dans la détection d'apolipoprotéine B.**

Un support selon l'Exemple 1, en polystyrène QE 716/20020 NJW lot 8C071 de BP Chimie, est enduit avec des concentrations de 1,25 - 2,5 - 5 et 10 µg/ml d'anticorps antiapolipoprotéines B. Ces supports sont dénommés, ci-après, Support A.

Les plaques de microtitration du commerce sont dénommées ci-après Support X.

La révélation de la réaction est réalisée à l'aide d'un conjugué avec la peroxydase et d'un chromogène avantageusement constitué par l'orthophényldiamine ; la lecture de l'absorbance étant réalisée au spectrophotomètre à 492 nm.

Les résultats obtenus dans ces conditions sont résumés dans les tableaux I et II ; ces résultats, pour une concentration donnée, sont la moyenne de 96 mesures.

La répétabilité est mesurée par le coefficient de variation ; elle est d'autant meilleure que le coefficient est faible. Le bruit de fond étant négligeable, on ne considère ci-après que les absorbances.

**Premier essai :**

TABLEAU I

| | SUPPORT A | | SUPPORT X | |
|---|---|---|---|---|
| concentration d'enduction en µg/ml | absorbance | coefficient de variation en % | absorbance | coefficient de variation en % |
| 1,25 | 0,321 | 8,4 | 0,235 | 9,5 |
| 2,50 | 0,742 | 6,0 | 0,587 | 13,9 |
| 5,0 | 1,002 | 6,6 | 0,857 | 12,3 |
| 10,00 | 1,185 | 5,1 | 1,046 | 10,3 |

**Deuxième essai :**

Il diffère du premier en ce que le matériau plastique utilisé pour la réalisation du support A, s'il est du polystyrène QE 716/20020 NJW, est un lot différent (lot 7F145).

Le support X, pris en comparaison a été prélevé sur le même lot que celui du premier essai.

TABLEAU II

| | SUPPORT A | | SUPPORT X | |
|---|---|---|---|---|
| concentration d'enduction en µg/ml | absorbance | coefficient de variation en % | absorbance | coefficient de variation en % |
| 1,25 | 0,332 | 7,0 | 0,227 | 9,9 |
| 2,50 | 0,775 | 6,2 | 0,581 | 9,4 |
| 5,0 | 1,007 | 3,9 | 0,875 | 6,7 |
| 10,00 | 1,147 | 4,3 | 1,106 | 8,7 |

La comparaison des tableaux I et II permet de voir la bonne reproductibilité des tests réalisés avec les Supports A ; en effet, les tests réalisés avec les supports A présentent moins de variations, moins de dispersions, une meilleure fiabilité et permettent une meilleure interprétation des résultats que les supports X.

**Exemple 4 : Comparaison d'une détection de meningocoques A au moyen d'un support conforme à l'Exemple 2 enduit d'anticorps antimeningocoques et au moyen d'un support du commerce :**

- un support selon l'Exemple 2 est enduit avec des concentrations de 0,15 à 10 µg/ml d'antimeningocoques A ; ces supports sont ci-après dénommés supports A,
- les supports du commerce sont dénommés support X.

TABLEAU III

| concentration d'enduction en µg/ml | SUPPORT A | | SUPPORT X | |
|---|---|---|---|---|
| | absorbance | coefficient de variation en % | absorbance | coefficient de variation en % |
| 0,15 | 0,235 | 9,1 | 0,042 | 8,9 |
| 0,30 | 0,302 | 8,2 | 0,123 | 9,0 |
| 0,60 | 0,318 | 7,5 | 0,204 | 9,0 |
| 1,25 | 0,264 | 7,0 | 0,282 | 8,4 |
| 2,5 | 0,212 | 3,4 | 0,333 | 8,5 |
| 5 | 0,161 | 4,3 | 0,274 | 6,5 |
| 10 | 0,132 | 3,9 | 0,202 | 3,9 |

Les résultats du Tableau III montrent que pour les concentrations d'enduction les plus faibles, on obtient, avec les supports A, une absorbance élevée, alors que pour les supports X, l'absorbance est plus faible à de telles concentrations. Il en résulte une sensibilité accrue des dosages utilisant comme supports, les supports A. De plus, en ce cas, l'utilisation de concentrations faibles en produit d'enduction présente un intérêt économique certain. On constate également qu'avec les supports A, les coefficients de variation sont plus faibles que ceux obtenus avec les supports X. Il en résulte une meilleure répétabilité des mesures faites avec les supports A.

**Exemple 5 : Etude de la conservation de l'activité du support solide conforme à l'invention :**

Un support dit A en polystyrène cristal à usage médical et biochimique (STYRON 648267 de DOW CHEMICAL, marque déposée) et traité au 2,4-dinitrophénol dans les mêmes conditions que dans l'Exemple 1.

Des anticorps antiapolipoprotéines B sont fixés à deux, quatre et six mois après le traitement des supports A.

Les supports X témoins, après stockage dans les mêmes conditions que les supports A sont enduits et révélés parallèlement.

TABLEAU IV

| concentration en µg/ml | | temp initial $T_0$ | | $T_0$ + 2 mois | | $T_0$ + 4 mois | | $T_0$ + 6 mois | |
|---|---|---|---|---|---|---|---|---|---|
| | | support A | support X | A | X | A | X | A | X |
| 1,25 | absorbance | 0,515 | 0,576 | 0,439 | 0,661 | 0,597 | 0,768 | 0,610 | 0,486 |
| | coefficient de variation en % | 6,2 | 8,6 | 5,1 | 7,6 | 5,2 | 6,8 | 7,4 | 8,2 |
| 2,50 | absorbance | 0,834 | 0,813 | 0,745 | 0,960 | 0,917 | 1,110 | 0,981 | 0,782 |
| | coefficient de variation en % | 4,0 | 8,5 | 3,4 | 7,6 | 4,5 | 6,4 | 4,5 | 5,8 |
| 5,0 | absorbance | 1,080 | 0,972 | 0,993 | 1,125 | 1,234 | 1,340 | 1,253 | 1,030 |
| | coefficient de variation en % | 2,0 | 4,3 | 3,2 | 3,7 | 4,3 | 6,0 | 4,6 | 5,5 |
| 10,0 | absorbance | 1,211 | 1,083 | 1,058 | 1,232 | 1,342 | 1,492 | 1,359 | 1,162 |
| | coefficient de variation en % | 2,8 | 3,8 | 3,0 | 3,5 | 3,1 | 5,2 | 3,4 | 4,8 |

Le Tableau IV montre que la répétabilité des tests utilisant les supports A (coefficient de variation faible) est stable au cours du temps.

**Exemple 6: Rôle de l'irradiation avant ou après le traitement chimique :**

Des supports en polystyrène cristal STYRON 648267 de DOW CHEMICAL (marque déposée) subissent différents traitements :

- les supports A sont traités au 2,4 dinitrophénol dans les mêmes conditions que celles de l'Exemple 1,
- les supports B sont irradiés par des rayons gamma à 2,5 Mrad,
- les supports C subissent, dans un premier temps, un traitement au 2,4 dinitrophénol, dans les mêmes conditions que celles de l'Exemple 1, puis dans un deuxième temps, sont irradiés de la même manière que les supports B,
- les supports D sont dans un premier temps, irradiés de la même manière que les supports B, puis dans un deuxième temps sont traités au 2,4 dinitrophénol dans les mêmes conditions que celles de l'Exemple 1.

Les différents supports A, B, C et D sont enduits d'anticorps antiapolipoprotéines B comme précisé dans l'Exemple 3.

TABLEAU V

| | A | | B | | C | | D | |
|---|---|---|---|---|---|---|---|---|
| concentration en µg/ml | absorbance | coeff de variation en % | absorbance | coeff de variation en % | absorbance | coeff de variation en % | absorbance | coeff de variation en % |
| 1,25 | 0,512 | 8,2 | 0,321 | 8,5 | 0,784 | 8,0 | 0,695 | 7,9 |
| 2,50 | 0,980 | 6,2 | 0,684 | 7,4 | 1,183 | 6,3 | 1,120 | 6,0 |
| 5,0 | 1,164 | 6,3 | 0,950 | 7,1 | 1,285 | 6,0 | 1,280 | 6,2 |
| 10 | 1,243 | 5,4 | 1,138 | 6,8 | 1,302 | 5,2 | 1,352 | 6,0 |

La répétabilité est mesurée par le coefficient de variation ; elle est d'autant meilleure que le coefficient est faible. Seuls les supports B ne subissent aucun traitement chimique conforme à l'invention ; ceux-ci pré sentent, notamment aux concentrations d'enduction faibles, des absorbances faibles. Les supports A, C et D (ayant subis un traitement chimique conforme à l'invention) présentent une absorbance élevée, donc une sensibilité de mesure supérieure à celle des supports B. Comme le montrent les résultats obtenus avec les supports C et D, il importe peu que l'irradiation soit réalisée avant ou après le traitement chimique.

Ainsi que cela ressort de ce qui précède, l'invention ne se limite nullement à ceux de ses modes de réalisation, de mise en oeuvre et d'application qui viennent d'être décrits de façon plus explicite ; elle en embrasse au contraire toutes les variantes que peuvent venir à l'esprit du technicien en la matière, sans s'écarter du cadre, ni de la portée de la présente invention.

**Revendications**

1°) Support solide à usage biologique, caractérisé en ce que ledit support solide présente une polarité de surface modifiée par la fixation sur ladite surface d'un composé chimique entraînant la formation de radicaux libres et/ou de chaînes carbonées aliphatiques dérivées de structures cycliques métastables, ledit composé chimique étant de formule I,

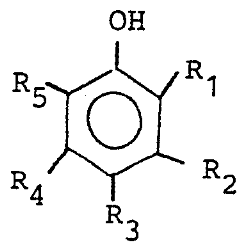

dans laquelle :
$R_1$ peut être un atome d'hydrogène, un groupe hydroxyle, un groupe $NO_2$, un halogène, un groupe alkyle inférieur comprenant 1 à 4 atomes de carbone, une amine ;
$R_2$ peut être un atome d'hydrogène, un groupe hydroxyle, un groupe $NO_2$, un halogène, un groupe alkyle inférieur comprenant 1 à 4 atomes de carbone, une amine ;
$R_3$ peut être un atome d'hydrogène, un groupe hydroxyle, un groupe $NO_2$, un halogène, un groupe alkyle inférieur comprenant 1 à 4 atomes de carbone, une amine, un groupe NO ;
$R_4$ peut être un atome d'hydrogène, un groupe $NO_2$, un halogène, un groupe hydroxyle ;
$R_5$ peut être un atome d'hydrogène, un groupe $NO_2$, un halogène, un groupe alkyle inférieur comprenant 1 à 4 atomes de carbone, un groupe hydroxyle.

2°) Support solide, selon la revendication 1, caractérisé en ce que, lorsqu'il est en plastique, il est choisi dans le groupe qui comprend les polystyrènes, les copolymères styréniques, le chlorure de polyvinyle, le polyméthacrylate de méthyle.

3°) Support solide selon la revendication 1 ou la revendication 2, caractérisé en ce que lorsque le composé chimique est du 2,4,5-trichlorophénol ou du 2,4,6- trichlorophénol, le support est avantageusement un copolymère styrénique.

4°) Support solide selon la revendication 1 ou la revendication 2, caractérisé en ce que lorsque le composé chimique est du 2-bromophénol, du 3-bromophénol ou du 4-bromophénol, le support est avantageusement choisi dans le groupe qui comprend les copolymères styréniques et le chlorure de polyvinyle.

5°) Support solide selon la revendication 1 ou la revendication 2, caractérisé en ce que lorsque le composé chimique est du iodophénol, le support chimique est avantageusement choisi dans le groupe qui comprend les copolymères styréniques, le chlorure de polyvinyle et le polyméthacrylate de méthyle.

6°) Procédé de traitement d'un support solide à usage biologique, en particulier dans le cadre de dosages immunologiques, caractérisé en ce que ledit support est mis en contact avec un composé chimique de formule I :

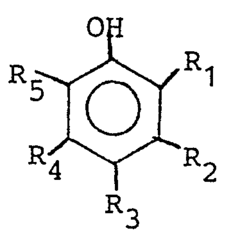

dans laquelle :

$R_1$ peut être un atome d'hydrogène, un groupe hydroxyle, un groupe $NO_2$, un halogène, un groupe alkyle inférieur comprenant 1 à 4 atomes de carbone, une amine ;
$R_2$ peut être un atome d'hydrogène, un groupe hydroxyle, un groupe $NO_2$, un halogène, un groupe alkyle inférieur comprenant 1 à 4 atomes de carbone, une amine ;
$R_3$ peut être un atome d'hydrogène, un groupe hydroxyle, un groupe $NO_2$, un halogène, un groupe alkyle inférieur comprenant 1 à 4 atomes de carbone, une amine, un groupe NO ;
$R_4$ peut être un atome d'hydrogène, un groupe $NO_2$, un halogène, un groupe hydroxyle ;
$R_5$ peut être un atome d'hydrogène, un groupe $NO_2$, un halogène, un groupe alkyle inférieur comprenant 1 à 4 atomes de carbone, un groupe hydroxyle.

7°) Procédé selon la revendication 6, caractérise en ce que le support solide est mis en contact avec ledit composé chimique pendant un temps approprié et à une température appropriée, puis lavé et séché.

8°) Procédé selon la revendication 6 ou la revendication 7, caractérisé en ce que le temps de contact est de 2 à 10 minutes.

9°) Procédé selon la revendication 7, caractérisé en ce que la température de traitement est comprise entre la température ambiante et 85° C.

10°) Procédé selon l'une quelconque des revendications 6 à 9, caractérisé en ce que le support solide est soumis à la fois à un traitement par ledit composé chimique et à un traitement d'irradiation, notamment par les rayons gamma.

11°) Procédé selon la revendication 10, caractérisé en ce que le traitement d'irradiation est réalisé préalablement à la mise en contact avec le composé chimique.

12°) Procédé selon la revendication 10, caractérisé en ce que le traitement d'irradiation est réalisé postérieurement à la mise en contact avec le composé chimique.

Office européen des brevets

# RAPPORT DE RECHERCHE EUROPEENNE

Numero de la demande

EP 89 40 0949

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 81, no. 1, 8 juillet 1974, page 352, résumé no. 4254f, Columbus, Ohio, US; R. KALIR et al.: "4-Hydroxy-3-nitrobenzylated polystyrene. Improved polymeric nitrophenol derivative for peptide synthesis", & EUR. J. BIOCHEM. 1974, 42(1), 151-6 * Résumé * | 1-3 | G 01 N 33/543<br>G 01 N 33/545 |
| A | EP-A-0 206 302 (CIBA-GEIGY) * Revendications 1-5; pages 4-5; page 8, lignes 9-20 * | 1-3 | |
| D,A | EP-A-0 061 167 (BEHRINGWERKE AG) * Page 2 * | 10-12 | |
| A | US-A-4 195 127 (F.J. HARTDEGEN) * Colonne 10, lignes 53-68; colonne 11, lignes 44-46 * | 1 | |

DOMAINES TECHNIQUES RECHERCHES (Int. Cl.4)

G 01 N
C 12 N
A 61 K

Le présent rapport a été établi pour toutes les revendications

| Lieu de la recherche | Date d'achèvement de la recherche | Examinateur |
|---|---|---|
| LA HAYE | 28-06-1989 | MEYLAERTS H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant